# EUROPÄISCHE PATENTSCHRIFT

(11) **EP 0 857 712 B1**
(45) Veröffentlichungstag und Bekanntmachung des Hinweises auf die Patenterteilung: **18.07.2001**
(21) Anmeldenummer: 97122246.8
(22) Anmeldetag: 17.12.1997
(51) Int. Cl.: C07C 51/09, C07C 61/04

(54) **Verfahren zur Herstellung von Cyclopropan-1,1-dicarbonsäure**
Process for the preparation of cyclopropane-1,1-dicarboxylic acid
Procédé pour la préparation d'acide cyclopropane-1,1-dicarboxylique

(30) Priorität: 06.02.1997 DE 19704449
(43) Veröffentlichungstag der Anmeldung: 12.08.1998
(73) Patentinhaber: Degussa AG, 40474 Düsseldorf (DE)
(72) Erfinder: Steffen, Klaus-Dieter, Dr., 53773 Hennef (DE)

(56) Entgegenhaltungen:
- EP-A- 0 571 299
- DE-A- 4 120 704
- DE-B- 1 155 780
- US-A- 2 373 011
- CHEMICAL ABSTRACTS, vol. 107, no. 25, 21.Dezember 1987 Columbus, Ohio, US; abstract no. 236059e, A. A. LARINA: "Improvement of technology for the manufacture of malonic acid" Seite 727; Spalte 1; XP002064491 & KHIM. TEKHNOL. (KIEV), Nr. 2, 1987, Seiten 11-12,
- M. K. BASU: "A mild and selective method of ester hydrolysis" SYNTHETIC COMMUNICATIONS, Bd. 19, Nr. 3&4, 1989, Seiten 627-631, XP002064490

## Beschreibung

Die Erfindung betrifft ein neues Verfahren zur Herstellung von Cyclopropan-1,1-dicarbonsäure der Formel 1 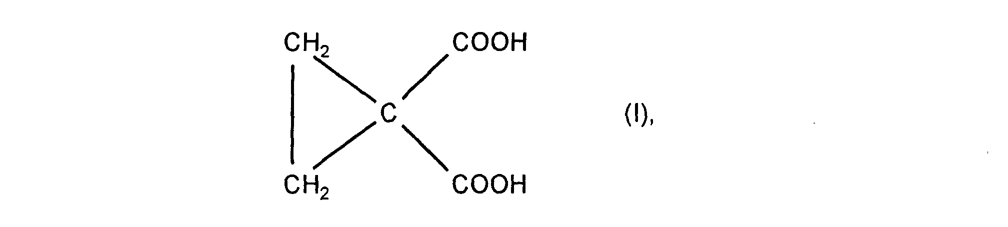 durch sauer katalysierte Verseifung der entsprechenden Methyl-, Ethyl-, Propyl-, Isopropyl- oder tert.-Butylester.

Malonsäure und Alkylmalonsäuren sind wichtige Zwischenprodukte für die Synthese von Agrochemikalien und Pharmawirkstoffen. Sie werden beispielsweise zur Herstellung von "Meldrum's Säuren", Barbituraten, Riechstoffen und Vitaminen eingesetzt.

Die Herstellung von Malonsäure und Alkylmalonsäuren wird allgemein in Kirk-Othmer, Encyclopedia of Chemical Technology, 3. Ed. (1981), Vol. 14, 794 bis 810, angegeben. Es kann eine saure oder alkalische Verseifung von Carbonsäureestern, -nitrilen oder -amiden angewandt werden. Dabei ist es oft ein Problem, dass die Produkte in Mischungen mit Alkalisalzen, z. B. Natriumchlorid, anfallen.

Erschwerend bei der Herstellung der Malonsäure und Alkylmalonsäuren ist ihre sehr gute Löslichkeit in Wasser und ihre leichte Decarboxylierung, die bei der Malonsäure bereits ab 70 °C einsetzt.

Die bekannteste Herstellung von Malonsäure und auch Methylmalonsäure geht von Chloressigsäure bzw. α-Chlorpropionsäure aus, die mit Natriumcyanid zu den entsprechenden Nitrilen umgesetzt und dann mit Natronlauge unter NH₃-Abspaltung verseift werden. Es folgt gemäß Org. Syntheses, Col. Vol. II (1943), 376, eine umständliche Isolierung über die Ca-Salze.

Wenn nach der alkalischen Verseifung eines Malonesters die Malonsäure als Alkalisalz gelöst in Wasser vorliegt, können die Alkali-Kationen nach DE-A-41 20 704 über saure lonenaustauscher entfernt und anschließend die freie Säure isoliert werden. Da bei alkalischen Verseifungen stets mindestens stöchiometrische Mengen an Alkali eingesetzt werden müssen, die bei der Malonsäure-Aufarbeitung wieder mit starken Säuren neutralisiert werden, fallen bei dieser Methode mindestens stöchiometrische Mengen an Salz an, die oft aufwendig entsorgt werden müssen. Das ist ein großer Nachteil dieser Methode der alkalischen Verseifung.

Bei der sauren Verseifung von Malonsäure-Derivaten werden anorganische Säuren in katalytischen Mengen zugesetzt. So kann die Verseifung von Diethylmalonat mit Schwefelsäure bei 70 °C zu Malonsäure erfolgen, wobei die Malonsäure durch Auskristallisation aus konzentrierter wässriger Lösung gewonnen werden kann. Unbefriedigende Ausbeuten durch Zersetzung der Malonsäuren in stark saurem Milieu und Korrosionsprobleme bei der Übertragung des Verfahrens in den großtechnischen Maßstab sind die Nachteile des Verfahrens durch saure Verseifung.

Cyclopropan-1,1-dicarbonsäure kann nach Org. Syntheses, Vol. 60 (1981), 66, aus Diethylmalonat, 1,2-Dibromethan, Natronlauge und stöchiometrischen Mengen eines Phasentransfer-Katalysators durch gleichzeitige Verseifung des intermediär gebildeten Cyclopropan-1,1-dicarbonsäurediethylesters in ca. 70%iger Ausbeute hergestellt werden. Neben der großen Menge an Phasentransfer-Katalysator, der 2,5-fachen Menge, bezogen auf die erhaltene Cyclopropan-1,1-dicarbonsäure, fallen auch beträchtliche Mengen an Natriumchlorid an, die - in Wasser gelöst - entsorgt werden müssen.

Dimethylmalonsäure ist ebenfalls lange bekannt und kann durch alkalische Verseifung des entsprechenden Diethylesters hergestellt werden. Die Verseifung des Dimethylesters mit Kalilauge zur Säure wird von W. Schauzer, K. Clusius, Z. physik. Chemie A 190 (1941), 243, ohne Nennung von Ausbeute und Reinheit erwähnt. Nach anderen Methoden wird die Säure durch Oxidation mit KMnO₄ oder HNO₃ von Methyl-Vorstufen gewonnen.

A. A. Larina, I. A. Tarkovskaya und G. K. Kamalov, Khim. Tekhnol. (Kiev) 1987 (2), 11-12, verseifen Malonester in wässriger Mischung an sulfonierten Kationenaustauschern zu Malonsäure, wobei kinetische Untersuchungen mit unterschiedlich vernetzten lonenaustauschern vorgenommen werden. Dabei wird angegeben, dass ein Wasser/Ethanol-Azeotrop destilliert und die Malonsäure aus Ethylacetat umkristallisiert wird. Werden dabei Ester und Wasser zusammen mit dem lonenaustauscher in einem Gefäß erhitzt, entsteht schwer filtrierbarer Abrieb an lonenaustauscher. Außerdem gibt es hier keine Hinweise auf Alkylmalonsäuren.

DE-B-1 155 780 beschreibt die Durchführung chemischer Reaktionen in Füllkörperkolonnen. Die Reaktion erfolgt dabei an mit lonenaustauschern überzogenen Füllkörpern. Als Beispiel einer Verseifungsreaktion wird die Herstellung von Bernsteinsäure aus Bernsteinsäuredimethylester und Wasser bei 100 °C beschrieben. Dabei wird Wasser in einem Kolben mit aufgesetzter Kolonne zum Sieder erhitzt und Bernsteinsäuredimethylester von oben auf den lonenaustauscher in der Kolonne gegeben. Dieses Verfahren liefert eine Bernsteinsäure, die noch mit erheblichen Mengen an Bernsteinsäuredimethylester verunreinigt ist. Außerdem ist die Herstellung des einzusetzenden lonenaustauschers aufwendig.

In EP-A-0 571 299 wird die gleiche Apparatur wie in DE-B-1 155 780 verwendet, wobei die Säule jedoch mit einem speziellen lonenaustauscher-Gel bestückt ist. Eine erhitzte Ester/Wasser-Mischung wird von oben auf das lonenaustauscher-Gel eingebracht. Das Verfahren ist zur Herstellung von Dicarbonsäuren mit 3 bis 6 C-Atomen geeignet, wobei Adipinsäure bevorzugt wird. Auch hier wird ein spezieller lonenaustauscher benötigt, der erhitztem Wasserdampf ausgesetzt wird. Cycloaliphatische Dicarbonsäuren werden nicht genannt.

Es bestand daher die Aufgabe, Cyclopropan-1,1-dicarbonsäure mit guten Ausbeuten und hoher Reinheit in einfacher Weise mit Hilfe von handelsüblichen lonenaustauscherperlen ohne störenden Salzanfall herzustellen, wobei das Verfahren umweltschonend und leicht in die Produktion übertragbar sein sollte.

Die Aufgabe wird erfindungsgemäß dadurch gelöst, dass man eine wässrige Mischung der Ester der Formel II 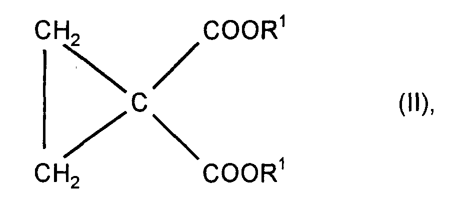 wobei R¹= CH₃, C₂H₅, C₃H₇ oder C(CH₃)₃ sein kann, durch ein Bett von sauren lonenaustauschern mit Sulfonsäure-Gruppen bei 30 bis 100°C und 40 bis 1 000 mbar leitet und dabei den entstehenden Alkohol abdestilliert, die erhaltene Cyclopropan-1,1-dicarbonsäure mit Hilfe von organischen Lösemitteln von Wasser befreit und dann durch Kristallisation in reiner Form gewinnt.

Da das Verfahren leicht verseifende Ester erfordert, kommen nur kurzkettige Alkylgruppen R¹ in Frage. Die Methylester verseifen sauer katalysiert am schnellsten. Sie werden dabei bevorzugt verwendet. Sie sind in der Regel auch die billigsten Ester.

Als saure Katalysatoren werden vernetzte Polystyrole, die sulfoniert worden sind, verwendet, wie sie als stark saure, Sulfonsäuregruppen enthaltende Kationenaustauscher verschiedener Hersteller im Handel zu erwerben sind (z. B. LEWATIT® S 100 und SP 112 der Bayer AG, D-51368 Leverkusen).

Wird der Katalysator direkt mit dem Ester und Wasser in den gerührten Reaktor eingebracht, so können die Katalysator-Perlen zum Teil mechanisch zerstört werden, was einen schlecht filtrierbaren, feinen Abrieb zur Folge haben kann.

Die sauren Austauscherharze werden daher in zylindrische Gefäße/Apparate . eingebracht und beidseitig (oben und unten) durch Siebe, Tücher, VLiese oder ähnliche, Flüssigkeit durchlässige Absperrungen eingeschlossen. Sie werden während der Reaktion meist von unten nach oben von dem aufgeheizten, wässrigen Medium durchströmt. Dabei destilliert der freigewordene Alkohol ab. Die wässrige Phase mit der gelösten Cyclopropan-1,1-dicarbonsäure wird vorzugsweise im Kreise geführt. Sie fließt in den beheizten Reaktor zurück.

Der lonenaustauscher-Katalysator kann beliebig oft wiederverwendet werden.

Für die Esterhydrolyse und als flüssiges Medium wird Wasser, in der Regel entionisiertes Wasser eingesetzt. Das Verhältnis der Mengen Ester (II) : Katalysator: H₂O kann in sehr weiten Grenzen variieren. Je nach Löslichkeit des Esters in Wasser - zu Anfang der Reaktion liegt in der Regel ein zweiphasiges Gemisch vor - wird die Wassermenge erhöht oder erniedrigt. Je größer die Katalysatormenge ist, desto schneller verläuft die Hydrolyse.

Wegen der thermischen Labilität der Cyclopropan-1,1-dicarbonsäure ist eine Reaktionstemperatur von 30 bis 100 °C bei einem Vakuum von 40 bis 1 000 mbar einzustellen.

Für die Isolierung der in Wasser gelösten Cyclopropan-1,1-dicarbonsäure bzw. für die Entfernung des Wassers mit Hilfe von organischen Lösemitteln werden zwei verschiedene Varianten bevorzugt angewandt:
1. Variante: Man destilliert 50 bis 95 % des ursprünglich eingesetzten Wassers ab. Die Destillation erfolgt vorzugsweise im Vakuum bei 40 bis 60 °C. Dann wird ein organisches Lösemittel zugesetzt, in dem die Cyclopropan-1,1-dicarbonsäure unlöslich ist, und das restliche Wasser azeotrop entfernt. Hier bieten sich solche Lösemittel an, die als Azeotrop höhere Mengen an Wasser enthalten, sich beim Kondensieren aber in 2 Phasen scheiden. Die wässrige Phase wird ausgeschleust. Geeignete Lösemittel sind Kohlenwasserstoffe und längerkettige Ether, wie z. B. Toluol, Cyclohexan und Dibutylether.
   Das abdestillierte Wasser kann bei Folgeansätzen wieder verwendet werden.
   Die Cyclopropan-1,1-dicarbonsäure, die aus dem organischen Lösemittel auskristallisiert, wird abfiltriert, gewaschen und im Vakuum getrocknet. Das Filtrat einschließlich Waschflüssigkeit des organischen Lösemittels kann direkt oder nach gelegentlicher Aufdestillation für Folgeansätze wieder eingesetzt werden.
   Gerade die Rückführung aller Lösemittel steigert die Ausbeute, wenn noch nicht oder nur einseitig hydrolysierte Ester vorhanden sind, die dann in den Folgeansätzen zu der Cyclopropan-1,1-dicarbonsäure verseift werden.
2. Variante: Die in Wasser gelöste Cyclopropan-1,1-dicarbonsäure wird mit einem organischen, nicht mit Wasser mischbaren Lösemittel, in dem die Cyclopropan-1,1-dicarbonsäure aber zum Teil löslich ist, extrahiert. Diese Extraktion muss wegen der guten Wasserlöslichkeit der Cyclopropan-1,1-dicarbonsäure mehrmals wiederholt werden. Hier werden vorzugsweise Ether eingesetzt. Geeignet sind dabei Ether mit 4 bis 7 C-Atomen, wie z. B. Methyl-t-butylether, Diethylether, Butylethylether oder Dipropylether. Das so extrahierte Wasser kann direkt wieder für Folgeansätze verwendet werden; dabei werden Energien für die Wasserdestillation gespart.

Das Extraktionslösemittel wird anschließend weitgehend abdestilliert, wobei auch Restwasser entfernt wird, worauf die Cyclopropan-1,1-dicarbonsäure schließlich auskristallisiert.

Für die Kristallisation kann man auch das hier erwähnte organische Lösemittel (z. B. Ether) der 2. Variante durch ein Nichtlösemittel (z. B. Toluol) der 1. Variante austauschen. Mit diesem Lösemitteltausch geht oft eine Reinigung und bessere Kristallisation der Cyclopropan-1,1-dicarbonsäure einher.

Die Reinheiten der nach dem erfindungsgemäßen Verfahren gewonnenen Cyclopropan-1,1-dicarbonsäure liegen oberhalb 99 %, die Ausbeuten bewegen sich zwischen 80 und 95 % d. Th.

Bei diesem Verfahren fällt als Nebenprodukt der frei gewordene Alkohol an, der anderweitig eingesetzt werden kann. Es entstehen sonst keine salzartigen oder wässrigen Abfallstoffe. Das Verfahren ist daher umweltschonend und leicht in den Produktionsmaßstab zu übertragen.

Für das Verfahren können auch die Halbester der Cyclopropan-1,1-dicarbonsäure eingesetzt werden.

Die folgenden Beispiele sollen die Erfindung verdeutlichen.

### Beispiel 1

### Herstellung von Cyclopropan-1,1-dicarbonsäure (CDS)

### a) Apparatur

Glas-Mehrhalskolben mit Doppelmantel zum Heizen und Bodenablaß, der an die Saugseite einer Schlauchpumpe angeschlossen ist. Die Druckseite der Pumpe führt zu einem Glasschuss, in dem sich der Katalysator befindet. Der Glasschuss mündet in den unteren Teil des Destillationsaufsatzes (einschließlich Kolonne), der auf dem Mehrhalskolben angebracht ist.

### b) Reaktionsdurchführung

In das Glasrohr der oben beschriebenen Apparatur werden 600 ml des lonenaustauschers LEWATIT® SP 112 (in der H⁺-Form) eingefüllt und beidseitig durch Fritten verschlossen. In dem Glaskolben befinden sich 500 g Cyclopropan-1,1-dicarbonsäuredimethylester (CDM, 98,8%ig, 3,12 Mol) sowie 750 ml entsalztes Wasser. Es wird bei einem Vakuum von 700 mbar zum Sieden erhitzt (89 bis 92 °C). Die Flüssigkeiten werden dabei aus dem Kolben über die lonenaustauscher-Säule im Kreis gepumpt. Bei einem Rücklaufverhältnis von 8 : 1 bis 15 : 1 destilliert Methanol bei 56 bis 58 °C ab. Nach 9 bis 10 Stunden ist die Verseifung beendet. Es wird abgekühlt und die Apparatur mit Wasser gespült. Die gesammelten wässrigen Lösungen werden nun mit 750 ml und zweimal mit 250 ml Methyl-t-butylether extrahiert. Der Ether wird weitgehend abdestilliert und durch 850 ml Toluol ersetzt. Der Ether wird nun restlos abdestilliert, wobei CDS auskristallisiert. Die Kristalle werden abfiltriert, mit Toluol gewaschen und getrocknet.

Auswaage und Analytik gehen aus Tabelle 1 hervor.

### Beispiele 2 und 3

### Herstellung von CDS

Es werden die gleichen Mengen an CDM wie in Beispiel 1 eingesetzt, und es wird der in Beispiel 1 benutzte Katalysator im Glasrohr wieder benutzt. Statt des entsalzten Wassers wird in Beispiel 2 die extrahierte wässrige Phase von Beispiel 1 und in Beispiel 3 die extrahierte wässrige Phase von Beispiel 2 verwendet. Die Durchführung der Verseifung erfolgt wie in Beispiel 1 beschrieben. In Beispiel 2 bzw. Beispiel 3 wird mit dem abdestillierten Methyl-t-butylether von Beispiel 1 bzw. Beispiel 2 extrahiert (Verluste werden jeweils durch frischen Methyl-t-buthylether ersetzt).

Die Toluol-Filtrate der Beispiele 1 und 2 werden aufdestilliert und deren Destillationssümpfe (41 g) im Beispiel 3 bei der Verseifung mit eingesetzt, da sie noch CDM und Cyclopropan-1,1-dicarbonsäure-monomethylester (CMS) enthalten.

Die Toluol-Destillate werden stets bei den nachfolgenden Ansätzen wiederverwendet.

Die Ergebnisse der Beispiele 2 und 3 befinden sich in nachfolgender Tabelle 1.

**Tabelle 1:**

| Beispiel | CDS | | Schmp. [°C] | Gehalt titr. | GC-Analyse [%] | | | H₂O [%] |
|---|---|---|---|---|---|---|---|---|
| | Auswaage | Ausbeute [% d.Th.] | | | CDS | CMS | CDM | |
| 1 | 320 | 77,9 | 138-139 | 99,9 | 98,93 | 0,06 | 0,06 | 0,15 |
| 2 | 360 | 87,4 | 137-138 | 99,3 | 98,65 | 0,11 | 0,06 | 0,31 |
| 3 | 410 | 99,5 | 137-138 | 99,1 | 98,61 | 0,07 | 0,05 | 0,15 |
| Summe | 1 090 | 88,4 | Ausbeuten alle mit 100 % Reinheit gerechnet | | | | | |

## Patentansprüche

1. Verfahren zur Herstellung von Cyclopropan-1,1-dicarbonsäure der Formel 1 durch sauer katalysierte Verseifung der zugehörigen Ester der Formel II nach dem Schema 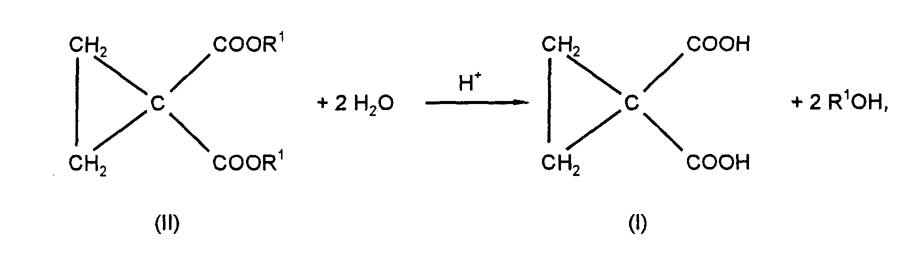 wobei
R¹ = CH₃, C₂H₅, C₃H₇, C(CH₃)₃
ist,
dadurch gekennzeichnet,
- dass man eine wässrige Mischung des Esters II durch ein Bett von sauren lonenaustauschern mit Sulfonäure-Gruppen bei 30 bis 100 °C und 40 bis 1 000 mbar leitet und dabei den entstehenden Alkohol abdestilliert,
- das Wasser mit Hilfe von organischen Lösemitteln von der Cyclopropan-1,1-dicarbonsäure abtrennt und dann
- die Cyclopropan-1,1-dicarbonsäure durch Kristallisation gewinnt.

2. Verfahren nach Anspruch 1,
dadurch gekennzeichnet,
dass R¹ = CH₃ ist.

3. Verfahren nach Anspruch 1,
dadurch gekennzeichnet,
dass man das Wasser zu 50 bis 95 % abdestilliert, ein organisches Lösemittel zusetzt und dann das restliche Wasser durch azeotrope Destillation entfernt.

4. Verfahren nach Anspruch 3,
dadurch gekennzeichnet,
dass als organisches Lösemittel Toluol, Cyclohexan oder Dibutylether verwendet wird.

5. Verfahren nach Anspruch 1,
dadurch gekennzeichnet,
dass man das Wasser dadurch abtrennt, dass man die Produkte mit einem organischen Lösemittel extrahiert.

6. Verfahren nach Anspruch 5,
dadurch gekennzeichnet,
dass man mit einem Ether mit 4 bis 7 C-Atomen extrahiert.

## Claims

1. A process for preparing cyclopropane-1,1-dicarboxylic acid of the formula I by acid-catalysed saponification of the associated esters of the formula II in accordance with the scheme 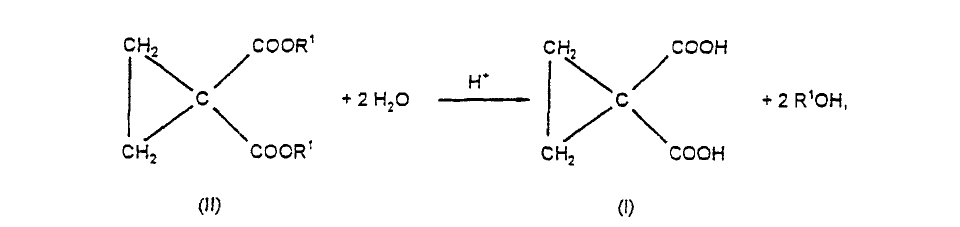 where
R¹ = CH₃, C₂H₅, C₃H₇, C(CH₃)₃,
characterized in that
- an aqueous mixture of the ester II is passed through a bed of acid ion exchanger containing sulphonic acid groups at from 30 to 100°C and from 40 to 1000 mbar, and the alcohol formed is distilled off,
- the water is separated, with the aid of organic solvent , from the cyclopropane-1,1-dicaboxylic acid, and then
- the cyclopropane-1,1-dicaboxylic acid is obtained by crystallization.

2. A process according to claim 1,
characterized in that
R¹ = CH₃.

3. A process according to claim 1,
characterized in that
from 50 to 95% of the water is distilled off, an organic solvent is added and the remaining water is then removed by azeotropic distillation.

4. A process according to claim 3,
characterized in that
toluene, cyclohexane or dibutyl ether is used as organic solvent.

5. A process according to claim 1,
characterized in that
the water is separated off by extracting the product with an organic solvent.

6. A process according to claim 5,
characterized in that
the extraction is carried out with an ether having from 4 to 7 C atoms.

## Revendications

1. Procédé de préparation d'acide cyclopropane-1,1-dicarboxylique de formule I par saponification catalysée par un acide, de l'ester correspondant de formule II selon le schéma : 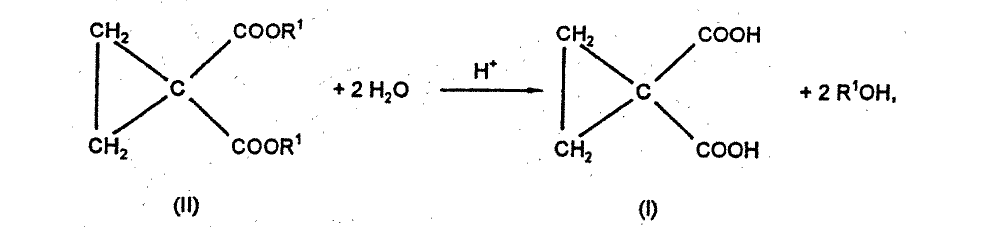 dans lequel R¹ est = CH₃, C₂H₅, C₃H₇ ou C(CH₃)₃,
caractérisé en ce qu'
- on conduit un mélange aqueux de l'ester II à travers un lit d'échangeurs d'ions acides ayant des groupes acide sulfonique, de 30 à 100°C et de 40 à 1000 mbars, et on sépare en même temps l'alcool qui s'est formé par distillation,
- on sépare de l'acide cyclopropane-1,1-dicarboxylique l'eau à l'aide de solvants organiques, et ensuite
- on récupère l'acide cyclopropane-1,1-dicarboxylique par cristallisation.

2. Procédé selon la revendication 1,
caractérisé en ce que
R¹ est = CH₃.

3. Procédé selon la revendication 1,
caractérisé en ce qu'
on sépare pour 50 à 95 % l'eau par distillation, on ajoute un solvant organique et on chasse l'eau résiduelle par distillation azéotropique.

4. Procédé selon la revendication 3,
caractérisé en ce qu'
on utilise comme solvant organique le toluène, le cyclohexane, ou l'éther dibutylique.

5. Procédé selon la revendication 1,
caractérisé en ce qu'
on sépare l'eau de telle sorte qu'on extrait les produits avec un solvant organique.

6. Procédé selon la revendication 5,
caractérisé en ce qu'
on extrait avec un éther ayant de 4 à 7 atomes de carbone.
